# EUROPEAN PATENT SPECIFICATION

(11) **EP 2 303 233 B1**
(45) Date of publication and mention of the grant of the patent: **20.06.2012**
(21) Application number: 08785086.3
(22) Date of filing: 25.07.2008
(51) Int. Cl.: A61K 9/16, A61K 47/12, A61K 9/20, A61K 9/28, A61K 9/26, A61K 9/48, A61K 31/4365

(54) **SOLID ORAL DOSAGE FORM CONTAINING ANTI-PLATELET AGENT CLOPIDOGREL AND METHOD FOR THE PREPARATION THEREOF**
DEN THROMBOZYTENAGGREGATIONSHEMMER CLOPIDOGREL ENTHALTENDE FESTE ORALE DOSIERFORM UND HERSTELLUNGSVERFAHREN DAFÜR
FORME GALÉNIQUE ORALE SOLIDE CONTENANT L AGENT ANTI-PLAQUETTAIRE CLOPIDOGREL ET SON PROCÉDÉ DE PRÉPARATION

(43) Date of publication of application: 06.04.2011
(73) Proprietor: Pharmathen S.A., 15351 Pallini Attikis (GR)
(72) Inventor: KOUTRIS, Efthimios, GR-153 51 Pallini Attikis (GR); SAMARA, Vicky, GR-153 51 Pallini Attikis (GR); KOUTRI, Ioanna, GR-153 51 Pallini Attikis (GR); ILIOPOULOU, Athina, GR-15351 Pallini Attikis (GR); BIKIARIS, Dimitrios, GR-543 51 Thessaloniki (GR)
(86) International application number: PCT/EP2008/006133
(87) International publication number: WO 2010/009745

(56) References cited:
- WO-A-2004/074215
- WO-A-2007/054968
- WO-A-2007/115305
- US-A1- 2007 048 370
- US-B1- 6 218 403

## Description

### TECHNICAL FIELD OF THE INVENTION

The present invention relates to improved solid dosage forms such as tablets and capsules and in particular to a pharmaceutical formulation for oral administration comprising a therapeutically effective quantity of an anti-platelet agent, and more particularly Clopidogrel or salt, derivative or polymorph thereof and a method for the preparation thereof.

### BACKGROUND OF THE INVENTION

Clopidogrel is a potent oral anti-platelet agent often used in the treatment of coronary artery disease, peripheral vascular disease, and cerebrovascular disease. Clopidogrel is indicated for prevention of vascular ischemic events in patients with symptomatic atherosclerosis, acute coronary syndrome without ST-segment elevation (NSTEMI), along with/without aspirin. It is also used, along with/without aspirin, for the prevention of thromboembolism after placement of intracoronary stent.

Clopidogrel, (+)-(*S*)-methyl 2-(2-chlorophenyl)-2-(6, 7-dihydrothieno [3, 2-*c*] pyridin-5 (4*H*)-yl) acetate, is a platelet aggregation inhibitor. The mechanism of action of clopidogrel is irreversible blockade of the adenosine diphosphate (ADP) receptor on platelet cell membranes. This receptor is named P2Y12 and is important in platelet aggregation, the cross-linking of platelets by fibrin. The blockade of this receptor inhibits platelet aggregation by blocking activation of the glycoprotein IIb/IIIa pathway. Platelet inhibition can be demonstrated two hours after a single dose of oral Clopidogrel, but the onset of action is slow, so that a loading-dose of 300-600 mg is usually administered.

Most consensus-based therapeutic guidelines recommend the use of Clopidogrel over aspirin, in patients requiring anti-platelet therapy but with a history of gastric ulceration, as inhibition of the synthesis of prostaglandins by aspirin (acetylsalicylic acid) can exacerbate this condition. A recent study has shown that in patients with healed aspirin-induced ulcers, however, patients receiving aspirin plus the proton pump inhibitor esomeprazole had a lower incidence of recurrent ulcer bleeding than patients receiving Clopidogrel.

Compositions containing thienopyridine derivatives, such as Clopidogrel or salts thereof are susceptible to degradation in alkaline environment. The degradation of the active ingredient results in reduced effectiveness and treatment failure. Several important problems that are associated with the nature of thienopyridine derivatives and in particular Clopidogrel or salts, derivatives or polymorphs thereof include heat and moisture sensitivity, tendency for decomposition and discoloration, as well as stability. The stability of pharmaceutical compositions containing Clopidogrel can also be influenced by the selection of the excipients.

Further, the tendency of thienopyridine derivatives, such as Clopidogrel or salts thereof to degrade may be accelerated by possible interactions with additional active ingredients or excipients present in the composition.

Thus, careful selection of excipients contributes to the maintenance of a stable composition for a long period of time by avoiding unwanted side effects that may arise from degradation of the drug.

Various methods are already known for the industrial preparation of oral dosage forms comprising thienopyridine derivatives, such as Clopidogrel or a pharmaceutical acceptable salt, derivative or polymorph thereof as an active ingredient due to its useful therapeutic properties. However, the prior art has encountered substantial difficulties in the production of solid oral formulations of desirable stability due to the degradation of said active ingredient.

Many compositions comprising bisulfate salts of Clopidogrel have been reported in the past and significant lower number of compositions comprising non-bisulfate salts, since these salts are more susceptible to degradation.

EP 1 310 245 discloses a tablet composition of Clopidogrel bisulfate and a lubricant selected from zinc stearate, sodium stearyl fumarate and stearic acid.

WO 2004/098593 discloses amorphous Clopidogrel hydrogen sulfate compositions comprising calcium stearate and/or magnesium stearate, a non hygroscopic additive and at least one excipient.

WO 2005/048992 discloses an oral administration form of Clopidogrel Mesylate, hydrobromide, hydroiodide or hydrochloride with a polymer coating selected from polyvinyl acetate or polyvinyl alcohol and a hydrogenated vegetable oil.

US 2007/0048370 discloses a tablet containing a salt of a monobasic acid with Clopidogrel or the solvates or hydrates thereof as active ingredient, except Clopidogrel hydroiodide, wherein the tablet contains no ionic and/or basic tableting excipient and no polyethylene glycol 6000.

Although each of the above patents represents an attempt to overcome the degradation and stability problems associated with pharmaceuticals compositions comprising a thienopyridine active ingredient such as Clopidogrel or salts, derivatives or polymorphs thereof, there still exists a need for improving the stability of such pharmaceutical compositions without unwanted pharmaceutical effects and with low production costs.

### SUMMARY OF THE INVENTION

It is, therefore, an object of the present invention to provide an improved solid dosage formulation for oral administration containing an anti-platelet agent and in particular Clopidogrel or pharmaceutical acceptable salt, derivative or polymorph thereof as an active ingredient, which overcomes the deficiencies of the prior art and enhances the stability of the composition.

Another object of the present invention is to provide a stable solid pharmaceutical dosage formulation for oral administration containing an anti-platelet agent and in particular Clopidogrel or pharmaceutical acceptable salt, derivative or polymorph thereof as an active ingredient, which avoids degradation of the active ingredient and is effective with sufficient self-life and good pharmacotechnical properties.

Another aspect of the present invention is to provide a solid dosage formulation for oral administration containing an anti-platelet agent and in particular Clopidogrel or pharmaceutical acceptable salt, derivative or polymorph thereof as an active ingredient, having a rapid and quantitive drug release.

A further aspect of the present invention is to provide a method for the preparation of a solid dosage formulation for oral administration containing an anti-platelet agent and in particular Clopidogrel or pharmaceutical acceptable salt, derivative or polymorph thereof as an active ingredient, thereby stabilizing said active ingredient and improving the pharmacotechnical characteristics of the composition.

In accordance with the above objects of the present invention, a pharmaceutical composition for oral administration is provided comprising an anti-platelet agent, and in particular Clopidogrel or pharmaceutical acceptable salt, derivative or polymorph thereof, as an active ingredient and an effective amount of an acidifying agent, such as citric acid as an agent to impart an acidic pH micro-environment in the dosage form and/or protect the active ingredient from degradation.

According to another embodiment of the present invention, a process for the preparation of a solid dosage forms for oral administration such as tablets, capsules and sachets, containing an anti-platelet agent, and in particular Clopidogrel or pharmaceutical acceptable salt, derivative or polymorph thereof as an active ingredient is provided, which comprises
- dissolving the total quantity of a binder such as hydroxypropyl cellulose (HPC) in water;
- forming a homogenous mixture by mixing the total quantity of said active ingredient with the total quantity of an effective amount of an acidifying agent such as citric acid as an agent to impart an acidic pH micro-environment in the dosage form, a portion of the total quantity of disintegrant such as crospovidone and optionally at least one pharmaceutically acceptable excipient such as a wetting agent, a diluent;
- kneading the above mixture with the binder solution and then drying the wetted mass;
- sieving the dried mass and adding to the sieved mixture the remaining portion of the total quantity of disintegrant and at least one optional excipient such as a lubricant and/or a glidant and mixing until uniform, and
- formulating the resulting mixture in a solid dosage form either by compressing it into a desired tablet form or filling capsules or sachets.

According to a further embodiment of the present invention, a process for the preparation of a solid dosage forms for oral administration such as tablets, capsules and sachets, containing an anti-platelet agent, and in particular Clopidogrel or pharmaceutical acceptable salt, derivative or polymorph thereof as an active ingredient is provided, which comprises:
- sieving the total amount of the active ingredient, at least one pharmaceutically acceptable excipient, and the total quantity of and an effective amount of an acidifying such as citric acid as an agent to impart an acidic pH micro-environment in the dosage form and/or protect the active ingredient form degradation, and forming a first blend;
- sieving at least one lubricant and blending with the first blend, and
- formulating the resulting mixture in a solid dosage form either by compressing it into a desired tablet form or by filling capsules or sachets.

Further preferred embodiments of the present invention are defined in dependent claims 2 to 10 and 13 to 15.

Other objects and advantages of the present invention will become apparent to those skilled in the art in view of the following detailed description.

### BRIEF DESCRIPTION OF THE DRAWINGS

Fig. 1 shows X-RD spectrum of active ingredient, placebo of composition 1A and composition 1A according to the present invention.
Fig. 2 shows dissolution profile of composition 1A with citric acid according to the present invention and composition 1B without citric acid having HCl 0.1N as dissolution medium
Fig. 3 shows X-RD spectrum of active ingredient, placebo of composition 3 and composition 3 directly after preparation and after 6 months stability according to the present invention

### DETAILED DESCRIPTION OF THE INVENTION

For the purposes of the present invention, a pharmaceutical composition comprising an anti-platelet agent, and in particular Clopidogrel or pharmaceutical acceptable salt, derivative or polymorph is considered to be "stable" if said ingredient degradates less or more slowly than it does on its own and/or in known pharmaceutical compositions.

An excipient is considered to be "incompatible" with an active ingredient (anti-platelet agent, and in particular Clopidogrel or pharmaceutical acceptable salt, derivative or polymorph thereof) if it promotes the degradation of said active ingredient, that is to say if said active ingredient (anti-platelet agent, and in particular Clopidogrel or pharmaceutical acceptable salt, derivative or polymorph thereof) degrades more or faster in the presence of said excipient when compared with degradation of said active ingredient on its own. The terms "incompatibility, "compatible" and "compatibility" are defined accordingly.

As already mentioned certain anti-platelet agents, such as Clopidogrel or pharmaceutical acceptable salts, derivatives or polymorphs thereof are susceptible to degradation during storage period and their tendency gets stronger when they are formulated and mixed with excipients and or other active substances.

It has been surprisingly found that the object of the present invention is achieved by employing an effective amount of an acidifying agent, such as citric acid in order to enhance the stability of the composition even after prolonged storage period.

Citric acid is widely used in pharmaceutical formulations and food products, primarily to adjust the pH of solutions. It has been used as an acidifying agent, antioxidant, buffering agent, chelating agent and flavouring agent. According to the present invention the active ingredient and the acidifying agent are in intimate contacting association in the composition and achieve optimal stability of the formulation.

The active ingredient (anti-platelet agent, and in particular Clopidogrel or pharmaceutical acceptable salt, derivative or polymorph thereof) and a suitable amount of citric acid are being admixed and subsequently wet granulated.

When citric acid is incorporated in a pharmaceutical composition according to the present invention, an acidic pH micro environment around the particles of the active ingredient is being formed, and thus decreasing the possibilities of degradation.

A salt of a monobasic acid with Clopidogrel or salts thereof is expected to be incompatible in a composition with alkaline compounds such as PEG 6000 and adversely affect the stability of the formulation. Surprisingly, the incorporation of citric acid according to the present invention, overcome this problem and provide stable compositions over prolonged storage time. By creating locally an acidic environment around the active substance, the occurrence of negative effect or reactions between the active ingredient and other alkaline excipients of the composition are minimized.

Moreover, any excipient may optionally be added to the above composition, provided that they are compatible with the active ingredient of the composition, in order to overcome problems associated with the unfavorable pharmacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms for oral administration of an active ingredient.

The manufacturing process for the preparation of the composition according to the present invention is simpler and inexpensive in comparison to any other conventional method.

Therefore, in a first embodiment, the present invention provides a pharmaceutical composition comprising from about 0.5% to 60% by weight of Clopidogrel or salt, derivative or polymorph thereof and from about 0.1 % to 5% by weight of citric acid. The weight ratio of the Clopidogrel or pharmaceutical acceptable salt, derivative or polymorph thereof to citric acid is preferably 1:10 to 600:1.

Preferred pharmaceutical compositions according to the present invention comprise Clopidogrel or salt, derivative or polymorph thereof in an amount approximately 1% to 50% and most preferably 5% to 50%.

More preferred pharmaceutical compositions according to the present invention comprise citric acid in an amount approximately 0.1% to 3%, and most preferably 0.1% to 2%.

The preferred pharmaceutical compositions are in the form of solid dosage forms for oral administration such as tablets, capsules, caplets, troches, pastilles, pills, lozenges and the like, in all shapes and sizes, coated or uncoated.

All percentages stated herein are weight percentages based on total composition weight, unless otherwise stated.

Another embodiment of the present invention is the use of the wet granulation process for the preparation of a solid dosage forms for oral administration comprising an anti-platelet agent, and in particular Clopidogrel or pharmaceutical acceptable salt, derivative or polymorph thereof as an active ingredient. Said wet granulation process comprises:
- dissolving the total quantity of a binder such as HPC in water;
- forming a homogenous mixture by mixing the total quantity of said active ingredient with the total quantity of an effective amount of an acidifying agent such as citric acid as an agent to impart an acidic pH micro-environment in the dosage form and improve the stability of the pharmaceutical composition, a portion of the total quantity of disintegrant such as crospovidone and optionally other pharmaceutically acceptable excipients such as a wetting agent, a diluent
- kneading the above mixture with the binder solution and then drying the wetted mass;
- sieving the dried mass and adding to the sieved mixture the remaining portion of the disintegrant and at least one optional excipient such as a lubricant and/or a glidant and mixing until uniform, and
- formulating the resulting mixture in a solid dosage either by compressing it into a desired tablet form or filling capsules or sachets.

A further embodiment of the present invention is the use of the direct compression process for the preparation of a solid dosage forms for oral administration comprising an anti-platelet agent, and in particular Clopidogrel or pharmaceutical acceptable salt, derivative or polymorph thereof as an active ingredient. Said dry granulation process comprises:
- sieving the total amount of the active ingredient, at least one pharmaceutically acceptable excipient, and the total quantity of and an effective amount of an acidifying such as citric acid as an agent to impart an acidic pH micro-environment in the dosage form and to improve the stability of the pharmaceutical composition, and forming a first blend;
- sieving at least one lubricant and blending with the first blend, and
- formulating the resulting mixture in a solid dosage form either by compressing it into a desired tablet form or by filling capsules or sachets.

Although the pharmaceutical composition may be in various forms, the preferred dosage form is solid such as tablets, capsules, minitablets incorporated in capsules, sachets and caplets.

Tableting is the preferred production method because it is faster, easier, adds fewer steps to the process and is the most economical. Further, the tableting method ensures a high production yield, contrary to the manufacture of pellets where the loss of production output is usually much higher. Excipients for the formulation are chosen carefully to give appropriate dissolution rate and pharmacotechnical properties to the finished dosage form.

The pharmaceutical compositions of the present invention may also contain one or more additional active agents such as acetylsalicylic acid, a lipid regulating agent such as a statin or an amino acid such as glycine.

The pharmaceutical compositions of the present invention may also contain one or more additional formulation ingredients selected from a wide variety of excipients as long as these excipients do not affect the stability of the composition. According to the desired properties of the composition, any number of ingredients may be selected, alone or in combination, based upon their known uses in preparation of solid dosage form compositions.

Such ingredients include, but are not limited to, diluents, binders, compression aids, disintegrants, surfactants, antioxidants, glidants, lubricants, flavours, water scavengers, colorants, sweetener, coating agents and preservatives.

Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch and sucrose.

Disintegrants may be, for example, alginic acid, carbon dioxide, carboxymethylcellulose calcium, carboxymethylcellulose sodium, microcrystalline cellulose, powdered cellulose, croscarmellose sodium, crospovidone, sodium docusate, guar gum, hydroxypropyl cellulose, methylcellulose, polacrilin potassium, poloxamer, povidone, sodium alginate, sodium glycine carbonate, sodium lauryl sulfate, sodium starch glycolate, starch, pregelatinized starch.

Surfactants may be, polyoxyethylene-polyoxypropylene co-polymers and block co-polymers, commercially available as Pluronic@ or Poloxamer@, ethoxylated cholesterins, commercially available as Solulan@, vitamin derivatives, e. g. vitamin E derivatives such as tocopherol polyethylene glycol succinate (TPGS), sodium dodecyl sulfate or sodium lauryl sulfate ; a bile acid or salt thereof, for example cholic acid, glycolic acid or a salt.

Glidants may be, for example, calcium silicate, powdered cellulose, starch, talc, colloidal silicon dioxide.

Lubricants may be polyethylene glycol 4000, polyethylene glycol 6000, sodium lauryl sulfate, starch, talc.

The following examples illustrate preferred embodiments in accordance with the present invention without limiting the scope or spirit of the invention:

### EXAMPLES

### EXAMPLE 1

**TABLE 1: Composition of example 1A**

| **Ingredients** | | **%** | **mg per tablet** |
|---|---|---|---|
| | **Internal phase** | | |
| **Clopidogrel** | | | **75,00** |
| **Clopidogrel Besylate** | | **44,84** | **112,10** |
| Microcrystalline cellulose | | **23,25** | 58,13 |
| Mannitol | | **20,16** | 50,40 |
| Hydroxypropylcellulose | | **2,00** | 5,00 |
| Crospovidone | | **2,50** | 6,25 |
| Citric acid | | **1,00** | 2,50 |
| | **External phase** | | |
| Crospovidone | | **2,50** | 6,25 |
| PEG 6000 | | **0,25** | 0,63 |
| Glyceryl behenate | | **3,00** | 7,50 |
| Talc | | **0,50** | 1,25 |
| | **Uncoated tablet** | **100,00** | **250,00** |

Tablets of the above formulation were prepared according to the following manufacturing process: HPC was dissolved in purified water. Clopidogrel besylate was mixed with Microcrystalline Cellulose, Mannitol, a portion of Crospovidone and citric acid to form a homogenous mixture. The above mixture was kneaded with the solution of HPC till a homogenous granule was created. The granular mass was dried and sieved. The remaining portion of Crospovidone and PEG 6000 were added to the dried granule and mixed. Finally, glyceryl behenate and talc were added to the mixture and mixed until complete homogeneity. The resulting granule was compressed into tablets, and optionally coated or compressed into mini tablets and filled in a capsule.

The produced tablets were tested for hardness, friability, disintegration and water content. All tests were performed according to European Pharmacopeia and were well within the specifications.

Composition 1B comprises the same ingredients as the above formulation but without comprising citric acid. Tablets according to Composition 1B were also prepared. The active ingredient of Composition 1A and Composition 1B was the besylate salt of Clopidogrel.

A commercially available tablet currently marketed as Plavix^{®} comprising the more stable salt, clopidogrel bisulfate, has been used as a reference composition (reference product).

Another object of the present invention was to prepare a pharmaceutical composition that is stable for a long period of storage time. Therefore, film- coated tablets of composition 1A, Composition 1B and the reference composition were exposed to normal (25°C±2°C/60%±5% RH) and accelerated (40°C±2°C/75%±5% RH) stability studies in closed vials according to the current ICH guidelines.

The stability results are shown in TABLE 2 and TABLE 3 below.

**TABLE 2: Stability results of Composition 1A, Composition 1B and reference composition directly after preparation and after 3 months of storage in normal conditions**

| **25°C/60 %RH CLOSED VIALS** | | | | | | |
|---|---|---|---|---|---|---|
| **SPECIFICATION** | **Composition** | **Composition** | **Reference product** | **Reference product** | **Composition** | **Composition** |
| | **(1A)** | **(1A)** | | | **(1B)** | **(1B)** |
| | **0 Months** | **3 Months** | **0 Months** | **3 Months** | **0 Months** | **3 Months** |
| **NMT 0.2%** | NA | 0,02% | 0.03% | 0.04% | 0.02% | 0.03% |
| **Imp D NMT 0.15%** | NA | NA | NA | NA | NA | NA |
| **NMT 0.3%** | 0.10% | 0,10% | NA | NA | 0.12% | 0.11% |
| **Unknown NMT 0.2%** | 0.02% | 0.02% | 0.02% | 0.04% | 0.09% | 0.05% |
| | 0.06% | 0.09% | 0.04% | 0.19% | - | 0.17% |
| | - | - | 0.10% | 0.02% | - | - |
| | - | - | 0.02% | - | - | - |
| | - | - | 0.02% | - | - | - |
| **Total NMT 1.5%** | **0,18%** | **0,23%** | **0.23%** | **0.29%** | **0.23%** | **0.36%** |

The stability results, as it is apparent from Table 2 and 3, indicate that the composition of the present invention (Composition 1A with citric acid) is remarkably more stable than the other two tested compositions.

**TABLE 3: Stability results of Composition 1A, Composition 1B and reference composition directly after preparation and after 3 months of storage in accelerated conditions.**

| **40°C/ 75 %RH CLOSED VIALS** | | | | | | |
|---|---|---|---|---|---|---|
| **SPECIFICATION** | **Composition** | **Composition** | **Reference product** | **Reference product** | **Composition** | **Composition** |
| | **(1A)** | **(1A)** | | | **(1B)** | **(1B)** |
| | **0 Months** | **3 Months** | **0 Months** | **3 Months** | **0 Months** | **3 Months** |
| **Imp A NMT 0.2%** | NA | 0.04% | 0.03% | 0.11% | 0.02% | 0.08% |
| **Imp D NMT 0.15%** | NA | NA | NA | NA | NA | NA |
| **Imp B NMT 0.3%** | 0.10% | 0.10% | NA | NA | 0.12% | 0.10% |
| **Unknown NMT 0.2%** | 0.02% | 0.04% | 0.02% | 0.03% | 0.09% | 0.06% |
| | 0.08% | 0.10% | 0.04% | 0.40% | - | 0.39% |
| | - | - | 0.10% | 0.09% | - | 0.96% |
| | - | - | 0.02% | - | - | 0.02% |
| | - | - | 0.02% | - | - | 0.03% |
| | - | - | - | - | - | 0.03% |
| | - | - | - | - | - | 0.07% |
| | - | - | - | - | - | 0.11% |
| | - | - | - | - | - | 0.04% |
| **Total NMT 1.5%** | **0.20%** | **0.28%** | **0.23%** | **0.63%** | **0.23%** | **1.89%** |

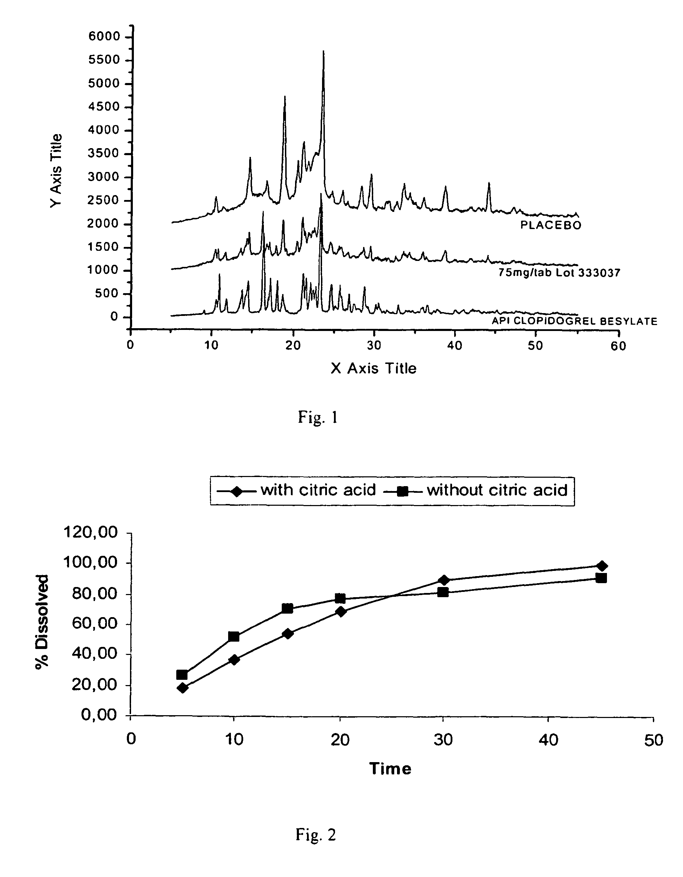

According to another aspect of the present invention, the active ingredient should remain in the same state after compression and should not convert in another form. As shown in Fig. 1 by the X-RD analysis of the active ingredient, placebo tablets and tablets of Composition 1A, Clopidogrel Besylate is in crystalline form and all the recorded characteristic main peaks (approximate 2θ values=10.75, 16.25, 17.10, 17.85) were unchanged after 3 months storage in both normal and accelerated conditions (40 °C and 75 % RH). The crystal properties remain also unchanged after three months in the same conditions when the mixture is incorporated in a pharmaceutical composition with other excipients. No peaks corresponding to another crystalline form of Clopidogrel Besylate are observed before or after storage indicating that the mixture is stabilized.

Another aspect of the present invention is to provide a solid dosage formulation for oral administration containing an anti-platelet agent and in particular Clopidogrel or pharmaceutical acceptable salt, derivative or polymorph thereof as an active ingredient, having a rapid and quantitive drug release.

Coated tablet dosage forms manufactured according to Composition 1A (with citric acid) and Composition 1B (without citric acid) were tested in an apparatus II dissolution tester, at 50 rpm having HCl 0.1N and HCl 0.01N as the dissolution medium.

As depicted from Fig. 2 and Table 4, the formulation prepared according to Composition 1A has indeed a quantitive and rapid release. The incorporation of citric acid to the composition besides the enhanced stability induces as well, the release of the active ingredient from the composition. Clopidogrel besylate used as the active substance in these compositions becomes less soluble as the pH value increases. Nevertheless, it's apparent from Table 4 that in both cases (two different pH values HCl 0.1N pH=1 and HCl 0.01N pH=2), the presence of citric acid increases the release.

**TABLE 4: Dissolution profiles of Composition 1A and Composition 1B under the same conditions in two different dissolution mediums**

| **Time** | **Composition (1B)** | **Composition (1A)** | **Composition (1B)** | **Composition (1A)** |
|---|---|---|---|---|
| | ***HCl 0.1N*** | | ***HCl 0.01N*** | |
| **5** | 26,28 | 17,68 | 20,72 | 27,40 |
| **10** | 51,42 | 36,58 | 41,53 | 60,09 |
| **15** | 70,35 | 54,49 | 60,63 | 82,32 |
| **20** | 77,28 | 68,84 | 74,59 | 87,83 |
| **30** | 81,22 | 89,57 | 78,63 | 90,34 |
| **45** | 91,35 | 99,42 | 80,76 | 91,78 |

### EXAMPLE 2

**TABLE 5: Composition of example 2**

| **Ingredients** | | **%** | **mg per tablet** |
|---|---|---|---|
| | **Internal phase** | | |
| **Clopidogrel** | | | **75,00** |
| **Clopidogrel Besylate** | | **44,84** | **112,10** |
| Microcrystalline cellulose | | **23,25** | 58,13 |
| Mannitol | | **20,16** | 50,40 |
| Hydroxypropylcellulose | | **2,00** | 5,00 |
| Croscarmellose sodium | | **2,50** | 6,25 |
| Citric acid | | **1,00** | 2,50 |
| | **External phase** | | |
| Crospovidone | | **2,50** | 6,25 |
| PEG 6000 | | **0,25** | 0,63 |
| Glyceryl behenate | | **3,00** | 7,50 |
| Talc | | **0,50** | 1,25 |
| | **Uncoated tablet** | **100,00** | **250,00** |

A main object of the present invention is to provide stable compositions upon prolonged period of storage time having quantitive and rapid release of the active ingredient. The incorporation of croscarmellose sodium as a disintegrant instead of crospovidone according to composition 1A of example 1 was tested (Table 5). The dosage forms of Composition 2 were prepared according to the manufacturing process of Composition 1A of example 1.

**TABLE 6: Stability results of Composition 2 directly after preparation and after 3 months of storage in normal and accelerated conditions**

| | | **25°C/60 %RH CLOSED VIALS** | **40°C/ 75 %RH CLOSED VIALS** |
|---|---|---|---|
| **SPECIFICATION** | **Composition 2** | **Composition 2** | **Composition 2** |
| | **0 Months** | **3 Months** | **3 Months** |
| **NMT 0.2%** | 0.02% | 0.09% | 0.06% |
| **Imp D NMT 0.15%** | NA | NA | NA |
| **Imp B NMT 0.3%** | 0.11% | 0.11% | 0.11% |
| **Unknown NMT 0.2%** | 0.05% | 0.29% | 0.65% |
| | - | 0.71% | 1.09% |
| | - | 0.05% | 0.09% |
| | - | 0.04% | 0.09% |
| | - | - | 0.11% |
| **Total NMT 1.5%** | **0,18%** | **1,29%** | **2.20%** |

**TABLE 7: Dissolution profiles of composition 1A (with crospovidone) and Composition 2 (with croscarmellose sodium) as disintegrant under the same conditions.**

| **Time** | **Composition 2** | **Composition 1A** |
|---|---|---|
| | ***HCl 0.1N*** | |
| **5** | 26,69 | 17,68 |
| **10** | 51,12 | 36,58 |
| **15** | 64,92 | 54,49 |
| **20** | 72,21 | 68,84 |
| **30** | 77,45 | 89,57 |
| **45** | 80,08 | 99,42 |

The stability results as shown in Table 6 in comparison with those in Tables 2 and 3, as well as the dissolution profile results (Table 7) indicate the superiority of composition as presented in example 1A with the use of crospovidone as a disintegrant.

### EXAMPLE 3

**TABLE 8: Composition of example 3**

| **Ingredients** | | **%** | **mg per tablet** |
|---|---|---|---|
| | **Internal phase** | | |
| **Clopidogrel** | | | **75,00** |
| **Clopidogrel Besylate** | | **44,84** | **112,10** |
| Microcrystalline cellulose | | **20,90** | 52,25 |
| Spray dried Mannitol (Mannogem^{™} EZ) | | **20,16** | 50,40 |
| Hydroxypropylcellulose | | **8,00** | 20,00 |
| Crospovidone | | **1,00** | 2,50 |
| Citric acid | | **1,00** | 2,50 |
| | **External phase** | | |
| PEG 6000 | | **0,10** | 0,25 |
| Glyceryl behenate | | **3,00** | 7,50 |
| Talc | | **1,00** | 2,50 |
| | **Uncoated tablet** | **100,00** | **250,00** |

Tablets of the above formulation were prepared according to the following manufacturing process: sieving and mixing until complete homogeneity the total quantities of Clopidogrel besylate, MCC, spray dried mannitol, crospovidone, HPC and citric acid. The total quantities of PEG 6000, glyceryl behenate and talc were sieved, then added to the above mixture and mixed until a homogenous granule is obtained. The granule can be filled in hard gelatin capsules or sachets or compressed into tablets, optionally coated or compressed into mini tablets and filled in a capsule.

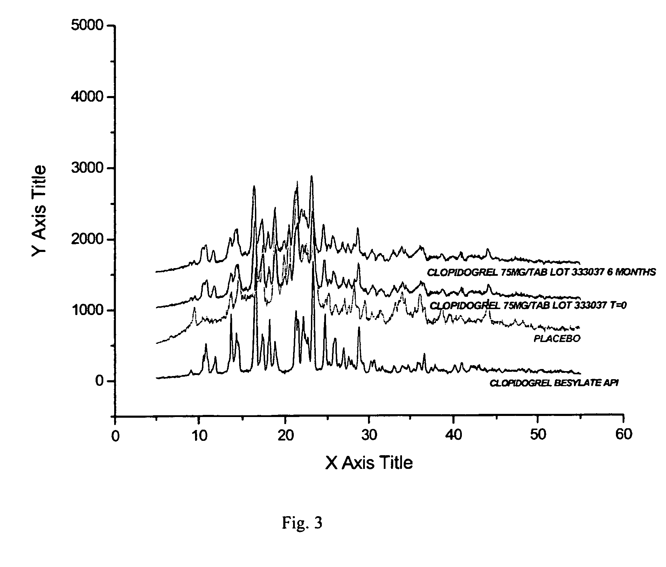

The pharmaceutical composition 3 of example 3 as it is apparent from Tables 9 and 10 below and the XRD of Fig. 3 does also concord with the main objects of the present invention. Composition 3 is a stable pharmaceutical dosage form after 6 months in normal and accelerated conditions and also has a rapid and quantitive release of the drug substance. From the XRD analysis it is depicted that the active ingredient in the tablets remains in the same crystalline form proving that the manufacturing process is appropriate for the purposes of the present invention

**TABLE 9: Stability results of Composition 3 directly after preparation and after 3 and 6 months of storage in blisters in normal and accelerated conditions.**

| | | **25°C/ 60 %RH TRIPLEX** | | **40°C/ 75 %RH TRIPLEX** | |
|---|---|---|---|---|---|
| **SPECIFICATION** | **Composition 3** | **Composition 3** | **Composition 3** | **Composition 3** | **Composition 3** |
| | **0 Months** | **3 Months** | **6 Months** | **3 Months** | **6 Months** |
| **Imp A NMT 0.2%** | 0.10% | 0.10% | 0.11% | 0.08% | 0.08% |
| **Imp D NMT 0.15%** | ND | ND | ND | ND | ND |
| **Imp B NMT 0.3%** | 0.05% | 0.05% | 0.05% | 0.06% | 0.07% |
| **Unknown NMT 0.2%** | | | | 0.05% | 0.06% |
| | | | | 0.17% | 0.18% |
| **Total NMT 1.0%** | **0.15%** | **0.15%** | **0.16%** | **0.36%** | **0.39%** |

**TABLE 10: Dissolution profile of composition 3 under the same conditions as composition 2 and composition 1A**

| **Time** | **Composition 3** |
|---|---|
| | ***HCl 0.1N*** |
| **5** | 24,82 |
| **10** | 52,46 |
| **15** | 79,42 |
| **20** | 90,11 |
| **30** | 98,11 |
| **45** | 100,06 |

The objects of the present invention are achieved by composition 3 employing an effective amount of an acidifying agent, such as citric acid in order to enhance the stability of the composition even after prolonged storage period. The selection of other appropriate pharmacologically acceptable excipients such as crospovidone and Mannogem EZ makes it possible to prepare the composition by using another manufacturing process than wet granulation.

The composition 3 of example 3 was prepared by the direct compression manufacturing process. In composition 3 Mannitol, a naturally occurring sugar alcohol found in animals and plants, was used. Mannitol is non hygroscopic, nonreducing hexahydric alcohol. It does not add moisture or contribute to moisture pickup. It is also chemically inert. These properties make Mannitol a useful excipient for tablets because it protects water-sensitive actives from degradation and does not react with the active.

Moreover, it has been found that Spray Dried Mannitol, selected form an extensive line of direct compressible Mannitol products gives comparative results. In addition Spray Dried Mannitol is a more specific diluent for direct compression than Mannitol granular. Specific tablets characteristics are much more improved with the use of Spray Dried Mannitol. This fact is justified by certain advantages of Spray Dried Mannitol over Mannitol granular, which are: the narrow particle size distribution and particle shape that allows it to be free flowing and easily mixed with other ingredients. Further, Spray Dried Mannitol is highly compressible, has sweet taste, smooth mouth feeling rapid disintegration properties.

These properties of Mannogem^{™} EZ (Spray Dried Mannitol) enable it to be used with high dose actives that may exhibit flow problems. Mannogem^{™} EZ Spray Dried Mannitol is highly compactable, non friable and quick dissolving.

The composition 3 of the present invention can therefore be considered, with respect the pharmacological performance, the best stable solid formulation currently available.

## Claims

1. A pharmaceutical composition for oral administration comprising anti-platelet agent Clopidogrel besylate as an active ingredient and an effective amount of citric acid, as an acidifying agent to impart an acidic pH micro-environment in the dosage form and/or protect the active ingredient from degradation, crospovidone as disintegrant, Spray Dry Mannitol, microcrystalline cellulose, polyethylene glycol 6000, hydroxypropyl cellulose, talc and a lubricant, wherein it comprises from about 0.5% to 60% by weight of Clopidogrel besylate and from about 0.1 % to 5.0% by weight of said acidifying agent

2. The pharmaceutical composition according to claim 1, wherein the weight ratio of said anti-platelet agent to the acidifying agent is preferably 1:10 to 600:1.

3. The pharmaceutical composition according to any preceding claim, wherein said composition is in solid dosage form such as tablet, capsule, sachet or mini tablets incorporated in a hard gelatin capsule.

## Patentansprüche

1. Eine pharmazeutische Formulierung zur oralen Verabreichung, enthaltend ein Anti-Thrombozyten-Mittel Clopidogrel Besylat als Wirkstoff und eine wirksame Menge der Zitronensäure, als ein Säuerungsmittel, um einen sauren pH-Mikro-Umgebung in der Dosierungsform verleihen und / oder zum Schutz der Wirkstoff vor Anbau, Crospovidon als Sprengmittel, Spray chemische Mannitol, mikrokristalline Cellulose, Polyethylenglykol 6000, Hydroxypropylcellulose, Talkum und einem Schmiermittel, wöbei es von etwa 0,5 bis 60 Gew.% von Clopidogrel-Besylat und von etwa 0,1 bis 5,0 Gew.% des Ansäuern Mittel.

2. Die pharmazeutische Formulierung gemäss Anspruch 1, **dadurch gekennzeichnet dass** das Gewichtsverhältnis des Anti-Thrombozyten-Agent Säuerungsmittel bevorzugt 1:10 bis 600:1

3. Die pharmazeutische Formulierung gemäss einem der vorangehenden Ansprüche, **dadurch gekennzeichnet dass** die Formulierung in fester Dosierungsform, wie Tabletten, Kapseln, Säckchen oder Minitabletten in einer Hartgelatinekapsel aufgenommen ist.

## Revendications

1. Une composition pharmaceutique pour administration orale comprenant l'agent antiagrégant plaquettaire: le Clopidogrel bésilate comme principe actif et une quantité efficace d'acide citrique, comme agent acidifiant pour conférer un micro-environnement de pH acide dans la forme posologique et/ou pour protéger le principe actif contre la dégradation, la crospovidone comme agent désintégrant, le mannitol pulvérisé à sec, la cellulose microcristalline, le polyéthylène glycol 6000, l'hydroxypropyle de cellulose, le talc et un lubrifiant, dans laquelle est compris d'environ 0.5% à 60% en masse de Clopidogrel bésilate et d'environ 0.1% à 5.0% en masse du dit agent acidifiant.

2. La composition pharmaceutique selon la revendication 1, dans laquelle le rapport en masse du dit agent antiagrégant plaquettaire et de l'agent acidifiant est préférablement de 1:10 à 600:1.

3. La composition pharmaceutique selon l'une quelconque des revendications précédentes, dans laquelle la dite composition est sous une forme posologique solide telle qu'un comprimé, une capsule, un sachet ou des mini-comprimés incorporés dans une capsule de gélatine dure.
